# EUROPÄISCHE PATENTANMELDUNG

(11) **EP 1 238 625 A2**
(43) Veröffentlichungstag der Anmeldung: **11.09.2002**
(21) Anmeldenummer: 02004712.2
(22) Anmeldetag: 01.03.2002
(51) Int. Cl.: A61B 1/307

(54) **Medizinisches Instrument zum Weiten der Harnröhre**

(30) Priorität: 09.03.2001 DE 20104228 U; 01.06.2001 DE 20109140 U
(71) Anmelder: Götz, Alois H., Dr., 91257 Pegnitz (DE)
(72) Erfinder: Götz, Alois H., Dr., 91257 Pegnitz (DE)
(74) Vertreter: Blaumeier, Jörg - Patentanwalt

(57) **Zusammenfassung**

Medizinisches Instrument zum Weiten der Harnröhre oder einer anderen Röhre oder Gefäßes des menschlichen oder tierischen Körpers, wobei das längliche Instrument (1) einen sich konisch im Durchmesser (D) erweiternden Aufweiteabschnitt (3) aufweist.

## Beschreibung

Die Erfindung betrifft ein medizinisches Instrument zum Weiten der Harnröhre oder einer anderen Röhre oder Gefäßes des menschlichen oder tierischen Körpers.

Derartige medizinische Instrumente üblicher Bauart (Nelaton, Tiemann) können nur eine mäßige, von der eingesetzten Kathetergröße abhängige Weitung erwirken. Daher müssen mehrere aufeinanderfolgende Katheterisierungen mit solchen Instrumenten oder Kathetern unterschiedlicher Größe vorgenommen werden, damit z.B. der Harnleiter auf den vom Arzt gewünschten Durchmesser geweitet werden kann. Dies ist jedoch aus mehrerlei Gründen nachteilig. Zum einen müssen mehrere Kathetergrößen stets vorgehalten werden, um die diskontinuierliche Mehrfach-Weitung vornehmen zu können. Hiermit ist eine entsprechende Belastung der Umwelt verbunden, da die Katheter als Sondermüll angesehen werden und entsprechend entsorgt werden müssen. Zum anderen müssen pro Aufweiteverfahren z.B. drei oder fünf Katheter verwendet werden, was das Verfahren insgesamt teuer macht. Darüber hinaus ist eine Mehrfachweitung zeitaufwendig. Am gravierendsten ist schließlich, dass der Patient die stufenweise Aufweitung mehrfach über sich ergehen lassen muss, was eine beachtliche körperliche Belastung dargestellt und unangenehm ist.

Der Erfindung liegt damit das Problem zugrunde, ein medizinisches Instrument anzugeben, das hier Abhilfe schafft.

Zur Lösung dieses Problems ist bei einem medizinischen Instrument der eingangs genannten Art erfindungsgemäß vorgesehen, dass das längliche Instrument einen sich konisch im Durchmesser erweiternden Aufweiteabschnitt aufweist.

Das erfindungsgemäße Instrument besitzt vorteilhaft einen sich im Durchmesser bevorzugt stufenlos vergrößernden Aufweiteabschnitt, der ein stufenloses Aufweiten z.B. der Harnröhre vom kleinsten Durchmesse des Aufweiteabschnitts bis hin zum maximalen Durchmessers des Aufweiteabschnitts zulässt. Die Harnröhre kann also in einem einzigen Katheterisierungsschritt auf den vom Arzt gewünschten Durchmesser aufgeweitet werden, indem der Katheter sachte Stück für Stück in die Harnröhre zur Weitung der selben eingeführt wird, bis der gesamte Aufweiteabschnitt eingeschoben ist und die maximale Aufweitung erreicht wurde.

Die hierdurch erzielbaren Vorteile liegen auf der Hand: Zum einen muss der Patient die Katheterisierung lediglich einmal über sich ergehen lassen, da die auf den vom Arzt gewünschten Weitungsgrad in einem Schritt erzielt werden kann und ferner muss pro Weitung nur ein einziger Katheter verwendet werden, was kostensenkend ist. Auch ist das ganze Verfahren wesentlich zügiger und kostengünstiger durchführbar, da nicht andauernd ein Instrumentenwechsel vorgenommen werden muss.

Der Durchmesser sollte sich wie beschrieben zweckmäßigerweise stufenlos vergrößern. Der Aufweiteabschnitt selbst kann erfindungsgemäß an oder nahe der Spitze des Instruments angeordnet sein. Nach einer ersten Erfindungsausgestaltung kann er unmittelbar an der Spitze des Instruments, diese quasi selber bildend angeordnet sein. Die Spitze kann dabei verschlossen oder geöffnet sein. Alternativ dazu kann der Aufweiteabschnitt auch im Anschluss an eine geschlossene separate Instrumentenspitze, die einige Millimeter oder Zentimeter lang ist, vorgesehen sein. Diese Spitze, die bevorzugt aus weichem Material ist und so ein besonderes leichtes Einführen in die Harnröhre zulässt, kann geradlinig, also in Achsrichtung verlaufend ausgeformt sein. Alternativ dazu besteht auch die besonders zweckmäßige Möglichkeit, die Spitze als Tiemann-Spitze auszubilden, an die sich der Aufweiteabschnitt anschließt. Eine solche Tiemann-Spitze ist bekannt, sie beschreibt eine leichte Krümmung oder Kurve und ist aus weichem Material. Ferner kann die Spitze jede andere gebogene oder auch geradlinig verlaufende Form aufweisen, wobei die jeweilige Form gegebenenfalls auf den jeweiligen Einsatzzweck des Instruments abgestellt sein kann.

Das Instrument selbst kann, gegebenenfalls mit Ausnahme der Spitze, hohl und rohrförmig sein, was es ermöglicht, z.B. wenigstens eine transparente oder offene Öffnung zur Sichtkontrolle der Röhre oder des Gefäßes, in die oder das das Instrument eingeführt ist, vorzusehen. Diese Öffnung kann im Bereich des hohlen Aufweiteabschnitts vorgesehen sein und ermöglicht es, die Harnröhre von innen auf etwaige Entzündungen oder Verletzungen oder dergleichen untersuchen zu können.

Weiterhin ist es zweckmäßig, wenn wenigstens eine Öffnung vorgesehen ist, durch die über das hohle Instrument zugeführte Flüssigkeit nach außen treten kann, um hiermit den Harnleiter oder dergleichen spülen zu können. Auch kann diese wenigstens eine Öffnung dazu dienen, dass Flüssigkeit durch sie in das Innere des Instruments eintreten kann oder aber, dass eine Gewebeprobe oder dergleichen in das Innere des Instruments geholt werden kann, wozu ein entsprechendes Biopsiebesteck durch das hohle Instrument bis durch die Öffnung hindurch zu schieben ist. Dies kann natürlich auch durch die bereits vorher beschriebene Öffnung erfolgen, es können aber auch mehrere Öffnungen vorgegeben sein.

Besonders zweckmäßig ist es, wenn wenigstens eine röntgenkontrastfähige Markierung am Instrument vorgesehen ist. Diese Markierung lässt es mit besonderem Vorteil zu, durch parallele Röntgenüberwachung die Position des Instruments in der Harnröhre kontinuierlich zu kontrollieren. Dabei kann die Markierung z.B. als an der Außenseite am Instrument, zweckmäßigerweise am Aufweiteabschnitt vorgesehene Markierung ausgebildet sein. Besonders zweckmäßig ist es aber, wenn die Markierung eine in der Spitze, insbesondere Tiemann-Spitze, vorgesehene Röntgenkontrastflüssigkeit ist, d.h., die Spitze ist mit dieser Röntgenkontrastflüssigkeit gefüllt.

An den im Wesentlichen starren Aufweiteabschnitt kann sich ein verlängernder schlauchartiger Abschnitt anschließen, der zweckmäßigerweise an den Aufweiteabschnitt angeklebt ist. Dieser schlauchartige katheterförmige und flexible Abschnitt verlängert also das katheterartige Instrument, so dass der Aufweiteabschnitt weit genug in das Gefäß oder die Röhre oder dergleichen eingeschoben werden kann.

Weiterhin kann vorgesehen sein, dass innerhalb oder außerhalb des schlauchartigen Abschnitts eine dehnbare, aufblasbare Membran, Blase oder ein Ballon vorgesehen ist, um im Bedarfsfall durch Aufblasen der Membran etc. eine Dilatation vorzunehmen.

Das Instrument selbst ist zweckmäßigerweise aus Kunststoff und vorzugsweise in einem Spritzgussverfahren hergestellt.

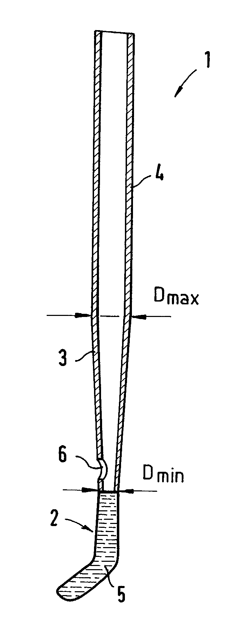

Weitere Vorteile, Merkmale und Einzelheiten der Erfindung ergeben sich aus dem im folgenden beschriebenen Ausführungsbeispiel sowie anhand der Zeichnung. Diese zeigt ein erfindungsgemäßes medizinisches Instrument 1, das im vorderen Bereich eine Tiemann-Spitze 2 aufweist, die bekanntermaßen eine gekrümmte Form aufweist. Im gezeigten Ausführungsbeispiel schließt sich an die weiche Tiemann-Spitze ein deutlich härterer Aufweiteabschnitt 3 an, der sich in seinem Durchmesser konisch erweitert, und zwar von einem kleinsten Durchmesser Dₘᵢₙ im unmittelbaren Anschlussbereich an die Tiemann-Spitze 2 bis zu einem maximalen Durchmesser Dₘₐₓ. Der maximale Durchmesser Dₘₐₓ definiert den Durchmesser, auf den der Harnleiter maximal aufgeweitet werden kann. Z.B. kann der minimale Durchmesser ca. 4,3 mm betragen und damit der genormten Charge 14 entsprechen. Der maximale Durchmesser Dₘₐₓ kann z.B. 7,2 mm und damit der genormten Charge 22 entsprechen. Es ist möglich, Dₘᵢₙ und Dₘₐₓ beliebig zu wählen. Je nachdem, wie groß der maximale vom Arzt gewünschte Weitungsgrad ist, wird der im Katheter mit dem entsprechenden maximalen Durchmesser Dₘₐₓ gewählt. Bei Harnleitern von Kindern ist natürlich ein kleinerer maximaler Durchmesser erforderlich, z.B. ein Durchmesser entsprechend genormter Charge 14. In diesem Fall wird der minimale Durchmesser Dₘᵢₙ der im Durchmesser der genormten Charge 8 entsprechen. Insgesamt ist die Wahl der minimalen und maximalen Durchmesser jedoch beliebig.

An den Aufweiteabschnitt 3 schließt sich ein verlängernder, schlauchförmiger Abschnitt 4 an, der wie auch der Aufweiteabschnitt 3 hohl ist. Der Abschnitt 4 kann beliebig lang sein.

Die Tiemann-Spitze 2 ist im gezeigten Ausführungsbeispiel mit einer Röntgenkontrastflüssigkeit 5 gefüllt, d.h., diese Flüssigkeit und damit die Instrumentenspitze kann in einer parallelen Röntgendurchleuchtung kontinuierlich in ihrer Position bestimmt und beobachtet werden. Die Spitze ist selbstverständlich geschlossen, so dass ein Austreten der Röntgenkontrastflüssigkeit 5 vermieden wird.

Am Aufweiteabschnitt 3 ist ferner eine Öffnung 6 vorgesehen, über die zum einen eine optische Sichtkontrolle des Leiterinneren möglich ist. Zum anderen kann hierüber aber auch eine über den Abschnitt 4 zugeführte Spülflüssigkeit austreten oder aber eine Gewebeprobe des Harnleiters oder dergleichen mittels eines über den hohlen Abschnitt 4 zugeführten Biopsiebestecks entnommen werden. Auch kann über diese Öffnung natürlich auch Flüssigkeit in das Instrument selbst eintreten und abgezogen werden.

## Patentansprüche

1. Medizinisches Instrument zum Weiten der Harnröhre oder einer anderen Röhre oder Gefäßes des menschlichen oder tierischen Körpers, **dadurch gekennzeichnet, dass** das längliche Instrument (1) einen sich konisch im Durchmesser (D) erweiternden Aufweiteabschnitt (3) aufweist.

2. Instrument nach Anspruch 1, **dadurch gekennzeichnet, dass** sich der Durchmesser (D) stufenlos ändert und vergrößert.

3. Instrument nach Anspruch 1 oder 2, **dadurch gekennzeichnet, dass** der Aufweiteabschnitt (3) an oder nahe der Spitze (2) des Instruments angeordnet ist.

4. Instrument nach Anspruch 3, **dadurch gekennzeichnet, dass** der Aufweiteabschnitt (3) unmittelbar an der Spitze des Instruments angeordnet ist.

5. Instrument nach Anspruch 3, **dadurch gekennzeichnet, dass** der Aufweiteabschnitt (3) im Anschluss an eine geschlossene Instrumentenspitze (2) vorgesehen ist.

6. Instrument nach Anspruch 5, **dadurch gekennzeichnet, dass** die Spitze als Tiemann-Spitze (2), als gebogen oder als gerade verlaufende Spitze ausgebildet ist, an die sich der Aufweiteabschnitt (3) anschließt.

7. Instrument nach einem der vorangehenden Ansprüche, **dadurch gekennzeichnet, dass** das Instrument (1), gegebenenfalls mit Ausnahme der Spitze (2) hohl und rohrförmig ist.

8. Instrument nach Anspruch 7, **dadurch gekennzeichnet, dass** wenigstens eine Öffnung (6) zur Sichtkontrolle der Röhre oder des Gefäßes, in die oder das das Instrument (1) eingeführt ist, vorgesehen ist.

9. Instrument nach Anspruch 8, **dadurch gekennzeichnet, dass** die wenigstens eine Öffnung (6) im Bereich des hohlen Aufweiteabschnitts (3) vorgesehen ist.

10. Instrument nach einem der Ansprüche 7 bis 9, **dadurch gekennzeichnet, dass** wenigstens eine Öffnung (6) vorgesehen ist, durch die über das hohle Instrument (1) zugeführte Flüssigkeit nach außen treten kann oder durch die Flüssigkeit in das Innere eindringen oder eine Gewebeprobe oder dergleichen in das Innere des Instruments (1) geholt werden kann.

11. Instrument nach einem der vorangehenden Ansprüche, **dadurch gekennzeichnet, dass** wenigstens eine röntgenkontrastfähige Markierung am Instrument (1) vorgesehen ist.

12. Instrument nach Anspruch 11, **dadurch gekennzeichnet, dass** die Markierung eine in der Spitze, insbesondere der Tiemann-Spitze (2) vorgesehene Röntgenkontrastflüssigkeit (5) ist.

13. Instrument nach einem der vorangehenden Ansprüche, **dadurch gekennzeichnet, dass** an den Aufweiteabschnitt (3) anschließend ein verlängernder schlauchartiger Abschnitt (4) anschließt.

14. Instrument nach Anspruch 13, **dadurch gekennzeichnet, dass** der verlängernde schlauchartige Abschnitt (4) an den Aufweiteabschnitt (3) angeklebt ist.

15. Instrument nach Anspruch 13 oder 14, **dadurch gekennzeichnet, dass** innerhalb oder außerhalb des schlauchartigen Abschnitts eine dehnbare, aufblasbare Membran, Blase oder ein Ballon vorgesehen ist.

16. Instrument nach einem der vorangehenden Ansprüche, **dadurch gekennzeichnet, dass** es aus Kunststoff ist.

17. Instrument nach Anspruch 16, **dadurch gekennzeichnet, dass** das den Aufweiteabschnitt und gegebenenfalls die Spitze aufweisende Instrumententeil in einem Spritzgussverfahren hergestellt ist.
